# EUROPEAN PATENT APPLICATION

(11) **EP 1 568 689 A1**
(43) Date of publication of application: **31.08.2005**
(21) Application number: 04003690.7
(22) Date of filing: 19.02.2004
(51) Int. Cl.: C07D 209/34, C07C 259/10

(54) **Process for the preparation of the 2-oxoindole derivative, Ropinirole**

(71) Applicant: Helm AG, 20097 Hamburg (DE); CF Pharma Gyogyszergyarto Kft., 1097 Budapest (HU)
(72) Inventor: Bertolino, Andrea, H-1145 Budapest (HU); Bor, Adam, H-2040 Budaörs (HU); Garaczi, Sandor, H-1048 Budapest (HU); Lukacs, Ferenc Dr., H-2143 Kistarcsa (HU); Orosz, György, H-1143 Budapest (HU); Schneider, Géza Dr., H-1097 Budapest (HU)
(74) Representative: Teipel, Stephan

(57) **Abstract**

The present invention relates to a process for the preparation of Ropinirole comprising the step of cyclizing a compound of the formula 15.

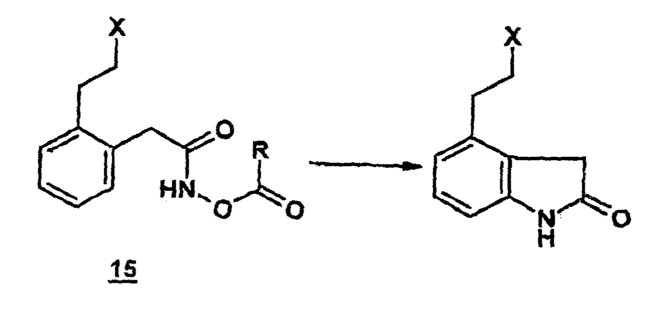

## Description

The present invention relates to an improved process for the preparation of 4-[2-(dipropylamino)ethyl]-1,3-dihydro-2H-indol-2-one (Ropinirole). This compound is described in EP-A-0 113 964 as being useful in cardiovascular therapy, and in EP-A-0 299 602 as agent useful in the treatment of Parkinson's disease based on its selective interaction with dopamine D₂-receptors.

EP-A-0 113 964 describes a process for the preparation of substituted indolinone derivatives, which comprises reduction by catalytic hydrogenation of a 2-nitrophenyl acetic acid precursor followed by spontaneous cyclization of the intermediate so formed. An improvement of this process by carrying out the reduction by transfer hydrogenation in water is described in EP-A-0 266 033. In industrial scale production of Ropinirole this reaction sequence is difficult to implement as 2-methyl-3-nitrophenyl acetic acid is required as starting material, which, however, is not commercially available. It can be obtained from o-toluic acid in five steps using expensive reagents like sodium borohydride. Moreover, the expensive and flammable borane is required in the selective reduction of an amide intermediate.

EP-A-0 300 614 discloses an alternative process for the preparation of substituted indolinone derivatives, which comprises reductive cyclization of 2-(2'-bromoethyl) β-nitrostyrene. This reaction route contains a step using Pd/C and in the reaction sequence for obtaining 2-(2'-bromoethyl) β-nitrostyrene a potentially dangerous bromination step is required.

An improvement to the reaction sequence disclosed in EP-A-0 300 614 is described in WO 91/16306 by replacing the halogen leaving group by a sulfonate group.

A further alternative process for the preparation of substituted indolone derivatives is disclosed in WO 94/15918. According to this document the indolone derivative is obtained by reduction of the corresponding isatin precursor compounds.

A problem of the present invention is to provide an improved process for the preparation of Ropinirole. In particular, the process provided should simplify the synthesis, for example by employing a synthetic route which does not contain dangerous or low-yield chemical steps. Furthermore, the process should use commercially available, cheap starting materials and reagents. A further aim is to avoid the use of Pd/C.

It has now unexpectedly been found that 4-substituted-2-indolinones can easily be obtained by cyclizing O-acyl hydroxamic acid derivatives.

Thus, the present invention relates to a process for the preparation of 4-[2-(dipropylamino)ethyl]-1,3-dihydro-2H-indol-2-one (Ropinirole), which comprises the step of cyclizing a compound of formula (15) or its acid addition salt 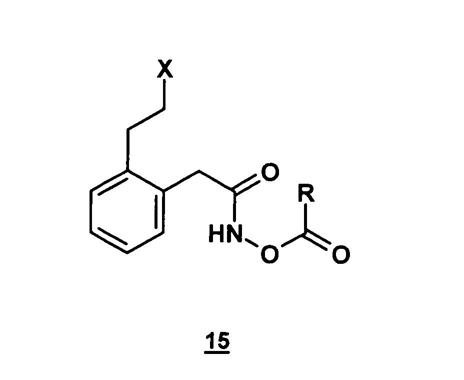 wherein
X is halogen, optionally protected hydroxy, optionally protected amino, alkyl amino or dialkyl amino; and
R is hydrogen, C₁₋₆ alkyl, C₃₋₆ allyl, phenyl, phenyl C₁₋₆ alkyl, C₁₋₆ alkoxy or phenyl C₁₋₆ alkoxy,
optionally removing any protective group from the cyclized derivative and, if necessary, converting the cyclized derivative into Ropinirole or its pharmaceutically acceptable salts.

In the above O-acyl hydroxamic acid derivatives of the formula (15) R can be hydrogen, C₁₋₆ alkyl, C₃₋₆ allyl, phenyl, phenyl C₁₋₆ alkyl, C₁₋₆ alkoxy or phenyl, C₁₋₆ alkoxy. The alkyl, allyl and alkoxy residues may be straight, branched or cyclic. Preferred residues R are methyl, t-butyl, phenyl and methoxy, particularly preferred is methyl.

X can be chosen from halogen, optionally protected hydroxy, optionally protected amino, alkyl amino and dialkyl amino. Herein halogen is for example chloro, bromo or iodo, preferably bromo. The hydroxy group may be optionally protected with any protective group known to the person skilled in the art as suitable for protecting hydroxy groups, e.g. alkyloxy, acyloxy, sulfonyloxy or silyloxy. The amino group may optionally be protected with any protective group known to the person skilled in the art as suitable for protecting amino groups, such as tert-butyloxycarbonyl, benzyloxycarbonyl, fluoroenylmethyloxycarbonyl, trifluoroacetyl, aryl or alkyl sulfonyl. The amino group may also comprise one or two alkyl groups, such as straight or branched C₁₋₆ alkyl. Preferably X is Dipropylamino.

The cyclization step in the process of the present invention can be conducted with the compound of the formula (15) or its acid addition salt, such as its hydrochloride. The cyclization is preferably carried out under Lewis acid catalyzed conditions, wherein the Lewis acid is preferably FeCl₃. In a preferred embodiment of the process of the present invention the molar ratio of the Lewis acid to the compound of formula (15) at the beginning of the cyclizing step is in the range of 1 : 0.1 to 1 : 10, preferably in the range of 1 : 1 to 1 : 5.

The cyclizing step can be carried out in any suitable solvent known to the person skilled in the art, such as halogenated and non-halogenated solvents, for example dichloromethane.

The temperature at which the cyclizing step is carried out is not of particular relevance and can be chosen by the skilled person. For example the cyclizing step can be conducted at a temperature between -15° and 150° C, preferably between 0° and 80° C, for example in dichloromethane under reflux.

If during the cyclizing step in the process of the present invention X in the compound of the formula (15) is a protected hydroxy or amino group, the protective group is removed from the cyclized derivative in a manner known to the skilled person. Moreover, if X in the compound of the formula (15) is not Dipropylamino, the cyclized derivative is converted into Ropinirole. Such conversion can be carried out by methods known to the person skilled in the art and being described for example in EP-A-0 300 614, WO 91/16306 and WO 94/15918. A possible synthesis route and examples of possible conversion routes are illustrated in the following scheme 1.

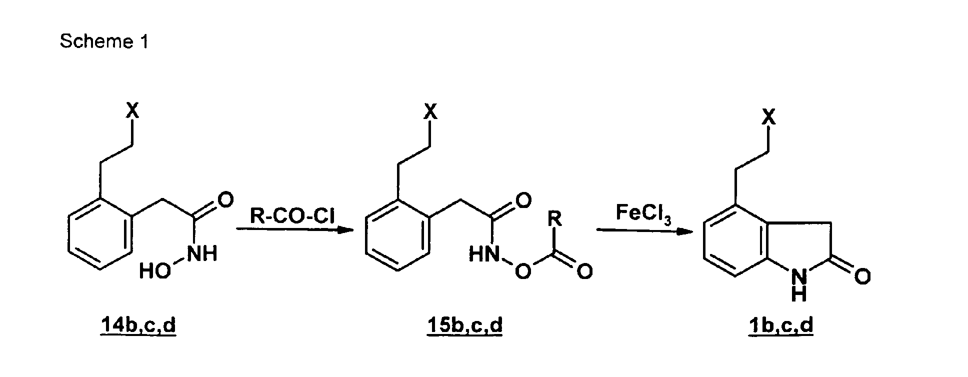

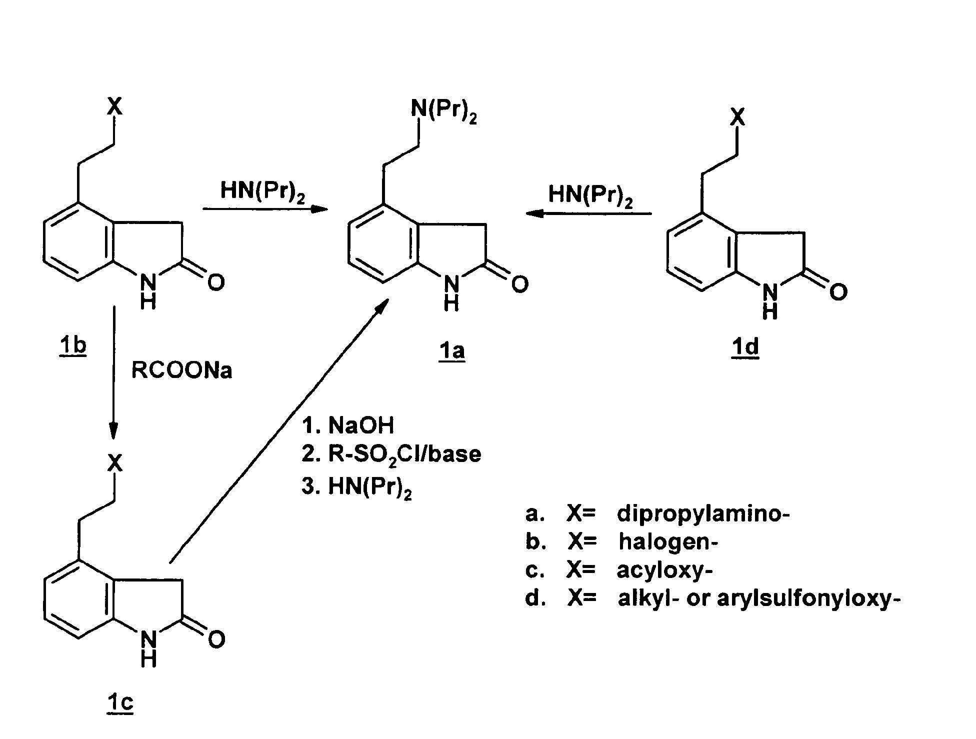

The O-acyl hydroxamic acid derivative of the formula (15) employed in the process of the present invention can be obtained by acylation of the corresponding hydroxamic acid derivative. Therefore, in a preferred embodiment of the process of the present invention the compound of the formula (15) is obtained by reacting a compound of the formula (14) 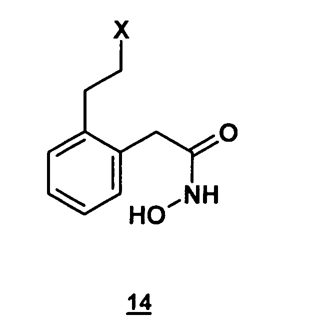 wherein X is defined as above with a compound of the formula

R-CO-Y

wherein R is defined as above and Y is a leaving group, for example Cl.

The hydroxamic acid derivative of the formula (14) according to a preferred synthetic approach can be efficiently synthesized according to the following scheme 2. In this approach the compound of the formula (14) is obtained from a cheap starting compound, 2-phenylethylalcohol (7). Compound (16) is also commercially available or can be synthesized according to the art. 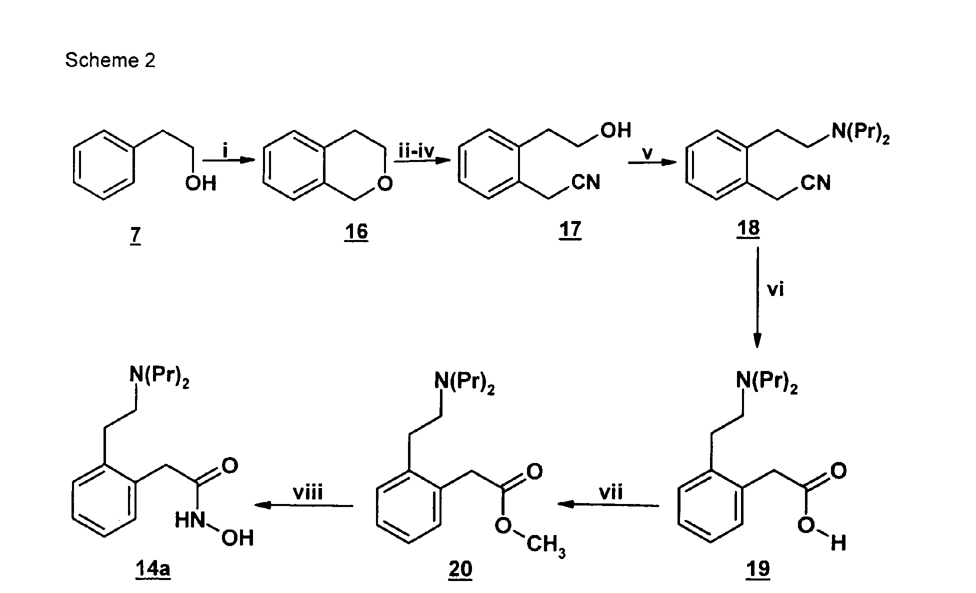
(i) (CH₂O)ₙ, HCl (ii) ZnCl₂, AcCl (iii) NaCN (iv) NaOH (v) CH₃-SO₂Cl, TEA, then NH(CH₂CH₂CH₃)₂ (vi) H₂SO₄/H₂O (vii) CH₃OH/HCl (viii) NH₂OH HCl, NaOH
   A particular advantage of the above synthetic route of scheme 2 is that the oxalate salt of the hydroxamic acid derivative (14) as well as the hydrochloride salt of the O-benzoyl derivative N-[(phenylacetyl)oxy]-2-{2-[2-(dipropylamino)ethyl]phenyl}acetamide are crystalline compounds which can be efficiently purified to obtain a compound of the formula (14) in high purity finally yielding Ropinirole of pharmaceutical grade in the last crystallization step.
   The specific conditions for carrying out the reactions according to above scheme 2 are known to the person skilled in the art. Moreover, for preparing the compound of the formula (17) selective ring opening methods of isochromane by several acyl chlorides and catalysts are described in the literature, e.g. with acetyl chloride and aluminium chloride (J. Colonge, P. Boisde, Bull. Soc. Chim. Fr. 1956, 1337) or by benzoyl chloride and zinc chloride (J.D. Hayler, S.L.B. Howie, R.G. Giles et al., J. Heterocyclic Chem. 32, 875 (1995)).
   Replacement of the halogen by cyanide and hydrolysis of the ester yields the nitrile alcohol of the formula (17).
   The compound of the formula (17) can be transformed either to a halogen derivative or preferably to the dipropylamino derivative of the formula (18). Subsequent hydrolysis (compound of the formula (19)), esterification (compound of the formula (20)), and finally reaction with hydroxylamine yields the appropriately substituted hydroxamic acid of the formula (14) as an oxalate salt which can be efficiently purified.
   The hydroxamic acid obtained can be acylated in a separate step to obtain the compound of the formula (15) or the acylation and cyclization steps can be performed as a one-pot reaction as demonstrated in examples 7 - 9.
   The quality of the Ropinirole obtained in the final cyclization step in the process of the present invention is high enough that the crude compound can be purified by conventional crystallizations to obtain a product which can be used as an active pharmaceutical ingredient.
   The invention is further illustrated by the following examples, which are not to be construed as limiting.

### Example 1: [2-(2-hydroxyethyl)phenyl]acetonitrile (17)

To the stirred solution of 316.9 g (1.49 mol) 2-(2-acetoxyethyl)benzyl chloride (prepared according to the procedure described in J. Colonge, P. Boisde, Bull. Soc. Chim. Fr. 1956, 1337) in 600 cm³ of dichloromethane a solution of sodium cyanide (87.64 g, 1.788 mol) in 160 cm³ of water and tetrabutyl ammonium bromide (4.804 g, 14.90 mmol) are added. The mixture is stirred at room temperature overnight.

10 M sodium hydroxide (298.0 cm³, 2.980 mol) and methanol (60 cm³) are added to the reaction mixture and the reaction is stirred at room temperature for 1 hour. The mixture is diluted with dichloromethane (400 cm³) and washed with water (400 cm³). The water phase is extracted with dichloromethane (2 x 200 cm³), the organic phases are mixed, washed again with water (3 x 500 cm³), dried over sodium sulfate, filtered and evaporated. The residue is crystallized from t-butyl methyl ether (400 cm³) to give 213.5 g (88.9%) of [2-(2-hydroxyethyl)phenyl]acetonitrile, melting between 53° and 55° C.

Elemental analysis: calcd. for C₁₀H₁₁NO (%): C, 74.51; H, 6.88; N, 8.69. Found: C, 74.83; H, 6.77; N, 8.48.

### Example 2: {2-[2-(dipropylamino)ethyl]phenyl}acetonitrile (18)

A mixture of 54.0 g (0.334 mol) of [2-(2-hydroxyethyl)phenyl]acetonitrile, 49.0 cm³ (0.351 mol) of triethyl amine, 27.2 cm³ (0.351 mol) of methanesulfonyl chloride and 108 cm³ of dichloromethane is stirred at 0° C for 10 minutes. After filtration, 40% sodium hydroxide (54 cm²) and dipropyl amine (91.85 cm³, 0.668 mol) are added and the solution is stirred at 60° C for 48 hours. The water phase is separated and washed with ethyl acetate (2 x 100 cm³). The combined organic phases are evaporated to remove dichloromethane and extracted with 5% hydrochloric acid. The combined acidic water extracts are turned basic by adding 40% sodium hydroxide to pH 10, extracted with dichloromethane (3 x 100 cm³), dried over sodium sulfate, filtered and evaporated to give 65.5 g (80.0%) of {2-[2-(dipropylamino)ethyl]phenyl}acetonitrile as a light yellow thick oil. Formation of the oxalic acid salt and recrystallization from isopropanol yields the oxalate salt as a white microcrystalline powder, m.p. 134° - 134.5° C.

Elemental analysis: calcd. for C₁₆H₂₄N₂·C₂H₂O₄ (%): C, 64.65; H, 7.84; N, 8.38. Found: C, 64.78; H, 7.90; N, 8.22.

### Example 3: {2-[2-(dipropylaminolethyl]phenyl}acetic acid (19)

A mixture of 10.0 g (0.352 mol) of {2-[2-(dipropylamino)ethyl]phenyl}acetonitrile, 20 cm³ of concentrated sulfuric acid and 10 cm³ of water is stirred at 100° C overnight. The pH of the reaction mixture is adjusted to pH 7 by adding 40% sodium hydroxide, concentrated by evaporation and extracted with dichloromethane (2 x 50 cm³), the combined organic extracts are dried over sodium sulfate, filtered and evaporated to give 10.67 g (99.1 %) of {2-[2-(dipropylamino)ethyl]phenyl}acetic acid as an amber-coloured oil.

### Example 4: methyl {2-[2-(dipropylamino)ethyl]phenyl}acetate (20)

A mixture of 6.36 g (24.14 mmol) of {2-[2-(dipropylamino)ethyl]phenyl}acetic acid, 10 cm³ of methanol and 2.64 cm³ of thionyl chloride (36.21 mmol) is stirred at room temperature overnight. The reaction is concentrated by evaporation, diluted with water, its pH adjusted to pH=8, extracted with dichloromethane (2 x 50 cm³), dried over sodium sulfate, filtered and evaporated to give 4.54 g (67.8%) of methyl {2-[2-(dipropylamino)ethyl]phenyl}acetate as a light yellow oil. Its oxalate salt can be recrystallized from isopropanol to give a white microcrystalline powder, melting at 121.5° - 122° C.

Anal. calcd. for C₁₇H₂₇NO₂·C₂H₂O₄ (%): C, 62.11; H, 7.96; N, 3.81. Found: C, 62.15; H, 7.99; N, 3.74.

### Example 5: 2-{2-[2-(dipropylamino)ethyl]phenyl}-N-hydroxyacetamide (14a)

A mixture of 97.02 g (0.350 mol) of methyl {2-[2-(dipropylamino)ethyl]phenyl}acetate, 60.75 g (0.874 mol) of hydroxylamine hydrochloride, 69.95 g (1.749 mol) of sodium hydroxide in 1000 cm³ of methanol and 350 cm³ of water is stirred for 0.5 hour. The reaction mixture is acidified and concentrated by evaporation. The residue is diluted with water, extracted with dichloromethane (3 x 250 cm³), dried over sodium sulfate, filtered and evaporated to give 82.25 g (84.5%) of 2-{2-[2-(dipropylamino)ethyl]phenyl}-N-hydroxyacetamide as an amber coloured syrup.

The oxalate salt formed in and recrystallized from isopropanol to give a white microcrystalline powder, characterized by a melting point of 152.5° - 153° C.

Elemental analysis: calcd. for C₁₆H₂₆N₂O₂·C₂H₂O₄ (%): C, 58.68; H, 7.66; N, 7.60. Found: C, 58.77; H, 7.73; N, 7.43.

### Example 6: N-[(phenylacetyl)oxy]-2-{2-[2-(dipropylmino)ethyl]phenyl}acetamide hydrochloride (15a, R = phenyl)

A mixture of 2.1 g (7.54 mmol) of 2-{2-[2-(dipropylamino)ethyl]phenyl}-*N*-hydroxyacetamide, 0.91 cm³ (7.92 mmol) of benzoyl chloride and 20 cm³ of dichloromethane is stirred at room temperature for 0.5 hour. After evaporation the residue is crystallized from ethyl acetate (10 cm³) to give 2.63 g (83.5%) of *N*-[(phenylacetyl)oxy]2-{2-[2-(dipropylamino)ethyl]phenyl}acetamide hydrochloride as a white microcrystalline powder, melting at 181 ° - 183° C.

### Example 7: Synthesis of 4-[2-(dipropylamino)ethyl]-1,3-dihydro-2H-indol-2-one hydrochloride (Ropinirole, 1a) from N-[(phenylacetyl)oxy]-2-{2-[2-(dipropylamino)-ethyl]phenyl}acetamide hydrochloride (15 a, R = phenyl)

A mixture of 2.09 g (5.00 mmol) of *N*-[(phenylacetyl)oxy]-2-{2-[2-(dipropylamino)ethyl]phenyl}acetamide hydrochloride, 3.24 g of anhydrous ferric chloride (20.0 mmol) and 15 cm³ of dichloromethane is stirred for 2 hours at reflux. The reaction mixture is evaporated, the residue suspended in minimal amount of dichloromethane and transferred to a silica column. Column chromatography with the eluent of methylene chloride : methanol : conc. NH₃ solution = 8 : 1 : 0.1 yields, after evaporation of the solvent, Ropinirole base as an oil. The evaporated residue is crystallized from isopropanol (5 cm³) and conc. HCl (0.5 cm³) to give 618 mg (41.7%) of 4-[2-dipropylamino)ethyl]-1,3-dihydro-2*H*-indol-2-one hydrochloride melting at 239° - 242° C.

IR (potassium bromide): 3417, 1759, 1724, 1703, 1614, 1597, 1456, 1242, 968, 795, 775;

¹H-NMR (500 MHz, deuterochloroform): 0.91 (t, 6H, J = 7.4 Hz, 12-CH₃), 1.71 (sex, 4H, J = 7.7 Hz, 11-CH₂), 2.98 (br t, 2H, 8-CH₂), 3.02 (m, 4H, 10-CH₂), 3.18 (br t, 2H, 9-CH₂), 3.54 (s, 2H, 3-CH₂), 6.72 (d, 1H, J = 7.7 Hz, 7-ArH), 6.84 (d, 1H, J = 7.7 Hz, 5-ArH), 7.13 (t, 1H, J = 7.8 Hz, 6-ArH), 10.42 (s, 1H, 1-NH), 10.84 (br, 1-H, NH⁺);

¹³C-NMR (500 MHz, deuterochloroform): 11.8 (q, C-12), 17.3 (t, C-11), 27.7 (t, C-8), 35.5 (t, C-3), 52.5 (t, C-9), 53.9 (t, C-10), 108.6 (d, C-7), 122.6 (d, C-5), 125.9 (s, C-3a), 128.7 (d, C-6), 134.0 (s, C-4), 144.7 (s, C-7a), 177.0 (s, C-2).

### Example 8: Synthesis of 4-[2-(dipropylamino)ethyl]-1,3-dihydro-2H-indol-2-one hydrochloride (Ropinirole, 1a) from O-benzoyl hydroxamic acid derivative formed in situ

A mixture of 2.970 g (10.67 mmol) of 2-{2-[2-(dipropylamino)ethyl]phenyl}-N-hydroxyacetamide, 1.305 cm³ (11.24 mmol) of benzoyl chloride and 25 cm³ of dichloromethane is stirred at room temperature for 0.5 hour. Anhydrous ferric chloride (5.192 g, 32.01 mmol) is added and the mixture is stirred for 2 hours at reflux. The reaction mixture is evaporated, the residue suspended in minimal amount of dichloromethane and transferred to a silica column. Column chromatography with the eluent of methylene chloride : methanol : conc. NH₃ solution = 8 : 1 : 0.1 yields after evaporation of the solvent Ropinirole base as an oil. This oil is crystallized from isopropanol (15 cm³) and conc. HCl (1 cm³) to give 800 mg (2.695 mmol, yield 25.3%) of 4-[2-(dipropylamino)ethyl]-1,3-dihydro-2H-indol-2-one hydrochloride melting at 239° - 242° C.

### Example 9: Synthesis of 4-[2-(dipropylamino)ethyl]-1,3-dihydro-2H-indol-2-one hydrochloride (Ropinirole, 1a) from O-acetyl hydroxamic acid derivative

A mixture of 11.18 g (40.16 mmol) of 2-{2-[2-(dipropylamino)ethyl]phenyl}-N-hydroxyacetamide, 3.00 cm³ (42.17 mmol) of acetyl chloride and 100 cm³ of dichloromethane is stirred at room temperature for 0.5 hour. Anhydrous ferric chloride (19.54 g, 0.120 mol) is added and the mixture is stirred for 2 hours at 50° C. The reaction mixture is evaporated, the residue suspended in minimal amount of dichloromethane and transferred to a silica column. Column chromatography with the eluent of methylene chloride : methanol : conc. NH₃ solution = 8 : 1 : 0.1 yields after evaporation of the solvent Ropinirole base as an oil. The evaporation residue is crystallized from isopropanol (50 cm³) and conc. HCl (4 cm³) to give 4.899 g (41.1%) of 4-[2-(dipropylamino)ethyl]-1,3-dihydro-2*H*-indol-2-one hydrochloride having a melting point of 239° - 242° C.

## Claims

1. Process for the preparation of 4-[2-(dipropylamino)ethyl]-1,3-dihydro-2H-indol-2-one (Ropinirole) which comprises the steps of cyclizing a compound of the formula (15) or its acid addition salt 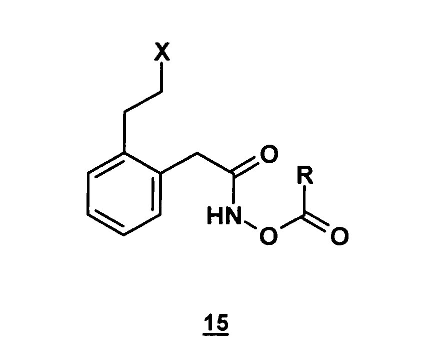 wherein
X is halogen, optionally protected hydroxy, optionally protected amino, alkyl amino or dialkyl amino; and
R is hydrogen, C₁₋₆ alkyl, C₃₋₆ allyl, phenyl, phenyl C₁₋₆ alkyl, C₁₋₆ alkoxy or phenyl C₁₋₆ alkoxy,
optionally removing any protective group from the cyclized derivative and, if necessary, converting the cyclized derivative into Ropinirole or its pharmaceutically acceptable salts.

2. Process according to claim 1, wherein the cyclizing step is carried out in the presence of a Lewis acid.

3. Process according to claim 2, wherein the Lewis acid is FeCl₃.

4. Process according to claim 2 or 3, wherein the molar ratio of the compound of formula (15) to the Lewis acid at the beginning of the cyclizing step is in the range of 1 : 0.1 to 1 : 10, preferably in the range of 1 : 1 to 1 : 5.

5. Process according to any of the proceeding claims, wherein the compound of formula (15) is obtained by reacting a compound of the formula (14) 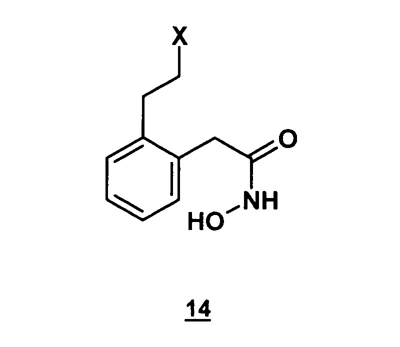 wherein X is defined as in claim 1 with a compound of the formula
R-CO-Y
wherein R is defined as in claim 1 and Y is a leaving group.

6. Process according to any of the proceeding claims, wherein R is methyl.

7. Process according to any of the proceeding claims, wherein X is Dipropylamino.

8. Compound of the formula (14) or its acid addition salt 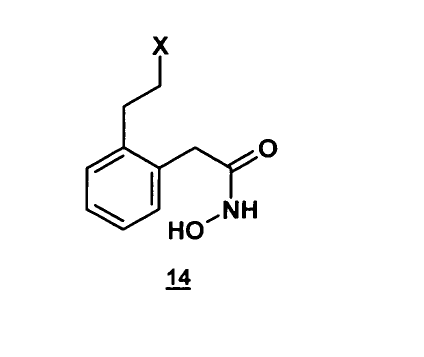 wherein X is halogen, optionally protected hydroxy, optionally protected amino, alkyl amino or dialkyl amino.

9. Compound according to claim 8, wherein X is Dipropylamino.

10. Compound of the formula (15) or its acid addition salt 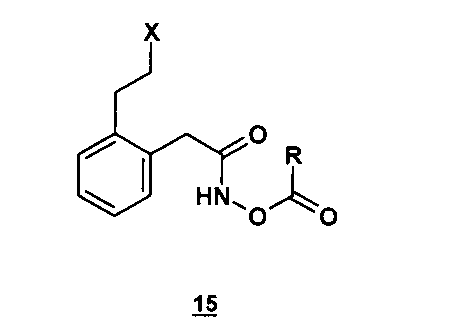 wherein
X is halogen, optionally protected hydroxy, optionally protected amino, alkyl amino or dialkyl amino; and
R is hydrogen, C₁₋₆ alkyl, C₃₋₆ allyl, phenyl, phenyl C₁₋₆ alkyl, C₁₋₆ alkoxy or phenyl C₁₋₆ alkoxy.

11. Compound according to claim 10, wherein X is Dipropylamino.

12. Compound according to claim 10 or 11, wherein R is methyl.

13. Use of a compound according to any of claims 8 - 12 for the preparation of Ropinirole or its pharmaceutically acceptable salts.
